# EUROPÄISCHE PATENTSCHRIFT

(11) **EP 2 208 476 B1**
(45) Veröffentlichungstag und Bekanntmachung des Hinweises auf die Patenterteilung: **20.07.2016**
(21) Anmeldenummer: 10158696.4
(22) Anmeldetag: 16.02.2006
(51) Int. Cl.: A61B 18/16, A61N 1/06

(54) **Flächige Elektrode**
Flat electrode
Electrode plate

(30) Priorität: 23.02.2005 AT 3062005
(43) Veröffentlichungstag der Anmeldung: 21.07.2010
(62) Teilanmeldung aus: 06704699.5
(73) Patentinhaber: Nessler Medizintechnik GmbH, 6020 Innsbruck (AT)
(72) Erfinder: Nessler, Norbert, 6020, Innsbruck (AT); Erbse, Stephan, 6063, Rum (AT)
(74) Vertreter: Dilg, Haeusler, Schindelmann Patentanwaltsgesellschaft mbH

(56) Entgegenhaltungen:
- EP-A- 0 463 196
- WO-A-00/65993
- CA-A1- 1 219 642
- DE-A1- 4 231 236
- US-A- 5 114 424

## Beschreibung

Die Erfindung betrifft eine flächige Elektrode, insbesondere Neutralelektrode, für medizinische Zwecke mit zwei gleich großen, entlang einer linienförmigen Isolierzone elektrisch voneinander getrennten Elektrodenflächen, die entlang einem inneren Randteilbereich unmittelbar aneinandergrenzen.

Unter flächigen Elektroden werden im Sinne der Erfindung alle medizinischen Elektroden zur Anbringung auf der Haut verstanden.

In der WO 00/65993 A ist in Fig.5 eine medizinische Neutralelektrode gezeigt, die zwei gleich große halbkreisförmige Elektrodenflächen aufweist, welche entlang einem inneren geradlinigen Randbereich einander gegenüberliegen und durch eine linienförmige Isolierzone voneinander getrennt sind. In der in Fig.8 dieser Druckschrift gezeigten Ausführungsform sind die beiden halbkreisförmigen Elektroden von einer ringartig um diese verlaufenden, bandartigen Elektrode umgeben, die vollkommen elektrisch isoliert von beiden Elektrodenhälften geführt ist. In Fig.3 dieser Druckschrift ist eine Elektrode gezeigt, bei der eine bandförmige Verlängerung einer Elektrodenhälfte sich um beinahe den gesamten restlichen inneren Teil der verschachtelten Elektrodenstruktur erstreckt.

Wie aus der DE 42 31 236 C2 bekannt, kann es während der elektrochirurgischen Behandlung zu einem teilweisen Abheben der Neutralelektrode von der Haut des Patienten und aufgrund der resultierenden stark erhöhten Stromdichte im Gewebe zu schweren Verbrennungen kommen, deren Auswirkungen am Patienten erst nach dem Aufwachen aus der Narkose bemerkt werden.

Um das vollflächige Anliegen der Neutralelektrode, sicherstellen zu können, sind Überwachungsvorrichtungen entwickelt worden, die während der Operation oder zwischen den einzelnen Behandlungsschritten Widerstands-oder Symmetriemessungen durchführen.

So verfügen die im Einsatz befindlichen Hochfrequenzgeneratoren üblicherweise über eine Contact Quality Monitoring-(CQM)-Einrichtung, welche unabhängig vom Schneidestrom der chirurgischen Apparatur die korrekte und vollflächige Applikation der Neutralelektrode auf der Haut des Patienten überprüft. Voraussetzung dafür sind zwei- oder mehrteilige Neutralelektroden, die eine Messung der Impedanz zwischen den Elektrodenteilen ermöglichen. Da der Hautwiderstand von Patient zu Patient stark variiert, ist eine eindeutige Aussage, ob die Neutralelektrode richtig aufliegt oder nicht oft nur sehr schwierig und es kann dabei zu Fehlmessungen kommen.

Durch geeignete Maßnahmen, wie z.B. durch eine möglichst lange linienförmige Isolierzone zwischen den Elektrodenteilen, wie dies in der DE 42 31 236 C2 beschrieben ist, lässt sich die Empfindlichkeit der Impedanzüberwachung steigern. Um auch ein distales Abheben der Elektrode erfassen zu können, wird die linienförmige Isolierzone mäandrierend über die gesamte Elektrodenfläche geführt. Eine flächige Ablösung der Elektrode wirkt sich somit annähernd proportional in der von der CQM-Vorrichtung gemessenen Impedanz aus.

Außerdem verfügen einige Hochfrequenzgeneratoren über eine Symmetrieprüfungs-Schaltung, welche über eine Messung des Ableitstromes der Elektrodenteile bestimmt, ob eine symmetrische Stromverteilung vorliegt oder ob ein Elektrodenteil einen höheren Stromanteil führt. Eine solche symmetrische Stromverteilung durch die Neutralelektrode kann mit Hilfe eines geeigneten Designs der Neutralelektrode gefördert werden. Wie sich aber gezeigt hat, reicht es nicht einfach aus, wie bei der Elektrode gemäß DE 42 31 236 C2 die Elektrodenteile mit gleicher Fläche zu dimensionieren, da die Stromaufnahme pro Flächeneinheit der Elektrode stark richtungsabhängig in Bezug auf das Operationsfeld und auch auf die Orientierung des umgebenden Muskelgewebes ist, die dem Fachpersonal bekannt ist.

Aufgabe der Erfindung ist es daher, eine eingangs genannte flächige Elektrode anzugeben, mit der über eine CQM-Proportionalisierung hinaus außerdem auch die Erzielung von symmetrischen Ableitströmen im Anwendungsbetrieb erreicht wird.

Erfindungsgemäß wird dies dadurch erzielt, dass die zwei gleich großen Elektrodenflächen jeweils einen bandförmigen Verlängerungsabschnitt aufweisen, welcher die jeweils andere Elektrodenfläche zumindest entlang einem äußeren Randteilbereich umgibt und dabei durch die verlängerte Isolierzone von der jeweils umgebenen Elektrodenfläche getrennt ist, und dass die beiden äußeren Randteilbereiche sich im wesentlichen jeweils ausgehend vom einen Ende des inneren Randteilbereiches um die jeweils andere Elektrodenfläche herum zumindest zum gegenüberliegenden anderen Ende des inneren Randteilbereiches erstrecken.

Auf diese Weise kann sowohl ein langer Isolierpfad durch die erfindungsgemäße Elektrode erreicht werden, der eine hohe Empfindlichkeit der CQM-Messung ermöglicht als auch eine Elektrodenflächengestaltung gewählt werden, die eine verlässliche Symmetriemessung der Ableitströme während der Operation gewährleistet, da auch in den Randbereichen, die einen überproportionalen Einfluss auf das Meßergebnis haben, die Anteile der Elektrodenfläche gleich groß angeordnet werden können.

Die zwei zentral gelegenen Elektrodenflächen sind dabei von den Verlängerungsabschnitten der jeweils anderen Elektrodenfläche umgeben. Ein relativ langer Isolierpfad und auch eine gegenüber den Randbereichen symmetrische Flächenaufteilung der Elektrodenflächen ist dadurch möglich.

Eine einfache konstruktive Gestaltung ergibt sich, wenn ein inneres Teilstück der linienförmigen Isolierzone und die aneinandergrenzenden inneren Randteilbereiche der zwei gleich großen Elektrodenflächen geradlinig verlaufen und sich entlang einer Nebenachse der flächigen Elektrode erstrecken.

Erfindungsgemäß ist die flächige Elektrode, insbesondere Neutralelektrode, für medizinische Zwecke mit zwei gleich großen, entlang einer linienförmigen Isolierzone elektrisch voneinander getrennten Elektrodenflächen, die entlang einem inneren Randteilbereich unmittelbar aneinandergrenzen, ausgestattet, wobei die zwei gleich großen Elektrodenflächen jeweils einen bandförmigen Verlängerungsabschnitt aufweisen, welcher die jeweils andere Elektrodenfläche zumindest entlang einem äußeren Randteilbereich umgibt und dabei durch die verlängerte Isolierzone von der jeweils umgebenen Elektrodenfläche getrennt ist, und wobei die beiden äußeren Randteilbereiche sich im wesentlichen jeweils ausgehend vom einen Ende des inneren Randteilbereiches um die jeweils andere Elektrodenfläche herum zumindest zum gegenüberliegenden anderen Ende des inneren Randteilbereiches erstrecken.

Um die Ausbildung von hohen lokalen Stromdichten während der Anwendung zu verhindern, sind gerundete Elektrodenformen von Vorteil, die kleinere Krümmungsradien in den Randbereichen vermeiden. Gemäß einer Weiterbildung der Erfindung können daher die zwei gleich großen Elektrodenflächen an ihrem dem inneren Teilstück der linienförmigen Isolierzone gegenüberliegenden Ende halbkreisförmig ausgebildet sein, welches Ende von den bandförmigen Verlängerungsabschnitten durch die äußeren Teilstücke der linienförmigen Isolierzone jeweils sichelförmig umgeben ist.

Für die Anwendung bei Symmetriestrom-Messungen ist eine hohe Flächensymmetrie der Einzelelektroden von Vorteil, In dieser Hinsicht kann ein Merkmal der erfindungsgemäßen Elektrode darin bestehen, dass die zwei Elektrodenflächen um eine Achse senkrecht zur Ebene der flächigen Elektrode zweifach-symmetrisch ausgebildet sind.

Weiters können Anschlusslaschen an einem Ende eines der Verlängerungsabschnitte der Flächenelektroden und einem Übergangsbereich zwischen der anderen der Flächenelektroden und des anderen Verlängerungsabschnittes angeordnet sein. Auf diese Weise kann das Anschließen der erfindungsgemäßen Elektrode an einer Seite erfolgen, wodurch die dieser Seite gegenüberliegende Seite, welche keine Anschlüsse aufweist, dafür geeignet ist, auf dem Patienten in Richtung des Operationsfeldes orientiert zu werden.

Eine vorteilhafte symmetrische Anordnung lässt sich erzielen, wenn die Anschlusslaschen sich an einer Seite der flächigen Elektrode in der gedachten Verlängerung des inneren Teilstückes entlang der Nebenachse erstrecken.

Weiters hat es sich aus mechanischen Gründen und aus Symmetriegründen als günstig erwiesen, wenn die Verlängerungsabschnitte sich jeweils in einem entlang der Nebenachse gelegenen Eckbereich der Flächenelektroden von diesen wegerstrecken, wobei die Eckbereiche gegenüber einer Hauptachse der flächigen Elektrode gleich beabstandet sind, und dass die Verlängerungsabschnitte sich jeweils um die andere der zwei Elektrodenflächen bis im wesentlich in die Nähe des Eckbereiches der anderen Elektrodenfläche erstrecken.

Eine andere Ausführungsform der Erfindung, bei der eine erhöhte Länge der Isolierzone vorliegt, kann darin bestehen, dass sich jeweils entlang der Hauptachse ein Vorsprung der bandförmigen Verlängerungsabschnitte in die jeweils andere Elektrodenfläche erstreckt.

Im zentralen Bereich der erfindungsgemäßen Elektrode ist die Stromdichte während der Anwendung relativ gering, sodass diese Zone dafür genutzt werden kann, Anschlüsse für die Elektrodenflächen vorzusehen. Dies kann dadurch erreicht werden, dass Anschlusslaschen im mittleren Bereich vorgesehen sind, die mit den Elektrodenflächen verbunden sind.

Diese Anbringung von Anschlusslaschen im zentralen Bereich einer flächigen Elektrode, insbesondere einer Neutralelektrode kann auch für alle bekannten Ausführungsformen dieses Elektrodentyps angewandt werden und wird als gesonderte Erfindung beansprucht.

Eine Vervielfachung des erfindungsgemäßen Prinzips, die innerhalb des Schutzumfangs der Erfindung liegt, kann beispielsweise dadurch erzielt werden, dass die um die jeweils andere Elektrodenfläche geführten Verlängerungsabschnitte anschließend in den Bereich der zugehörigen Elektrodenfläche zurückgeführt sind und dort, getrennt durch die linienförmige Isolierzone, die jeweils anderen Verlängerungsabschnitte umgeben.

Diesbezüglich sind weitere andere Formen der Führung der Verlängerungsabschnitte im Rahmen der Erfindung ausführbar. So kann vorgesehen sein, dass die bandförmigen Verlängerungsabschnitte um die beiden Elektrodenflächen spiralförmig ineinanderverlaufend zwei oder mehrfach herumgeführt sind.

Bei mittiger Anordnung der Anschlusslaschen, wenn diese also direkt mit den beiden zentral angeordneten Elektroden verbunden sind, kann es vorteilhaft sein, wenn der Stromanteil des inneren Bereiches gegenüber jenem des äußeren so beeinflusst wird, dass die sonst vorhandene Stromkonzentration in den Randbereichen der Elektrode sich gleichmäßiger über die gesamte Elektrodenfläche verteilt. Dies kann z.B. durch Vorsehen eines von außen nach innen gerichteten Impedanzgradienten erreicht werden. d.h. dass je weiter der Strom in den Außenbereich der Elektrode fließt sich die Impedanz für ihn erhöht.

Gemäß einer Ausführungsform der Erfindung können zu diese Zweck entlang des Verlaufes der bandförmigen Verlängerungsabschnitte diese an einer oder mehreren Stellen unterbrochen und Impedanzen zur Ausbildung eines Impedanzgradienten zwischengeschaltet sein.

Nachstehend wird die Erfindung anhand der in den Zeichnungen gezeigten Ausführungsbeispiele eingehend erläutert. Es zeigt dabei
Fig.1 eine schematische Draufsicht auf eine Ausführungsform der erfindungsgemässen Elektrode;
Fig.2 eine schematische Draufsicht auf eine weitere Ausführungsform der erfindungsgemässen Elektrode;
Fig.3 eine schematische Draufsicht auf eine weitere Ausführungsform der erfindungsgemässen Elektrode;
Fig.4 eine schematische Draufsicht auf eine weitere Ausführungsform der erfindungsgemässen Elektrode;
Fig.5 eine schematische Draufsicht auf eine weitere Ausführungsform der erfindungsgemässen Elektrode und
Fig.6 eine schematische Draufsicht auf eine weitere Ausführungsform der efindungsgemässen Elektrode.

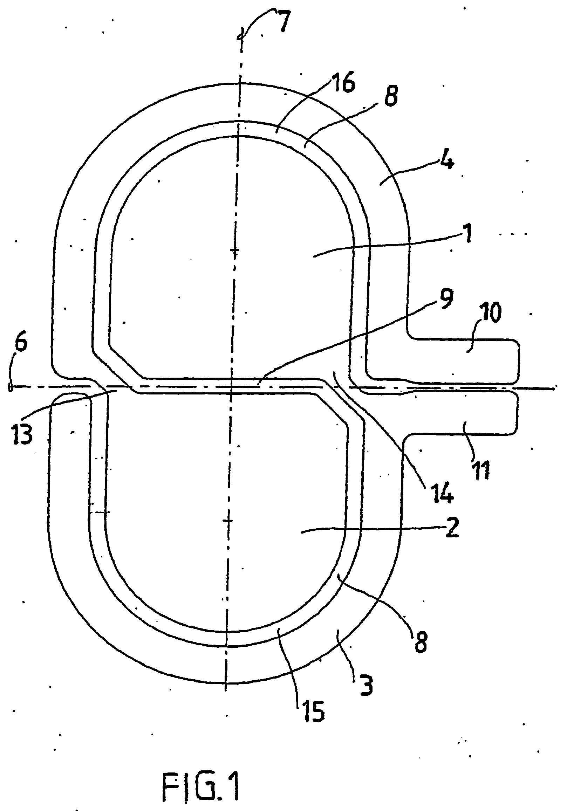

Fig.1 zeigt eine flächige Elektrode, insbesondere eine Neutralelektrode für die Hochfrequenz-Chirurgie. Die beiden durch ausgezogene Linien umrandeten Flächen sind zwei Elektrodenflächen 1, 2, die entlang einer linienförmigen Isolierzone 8 elektrisch voneinander getrennt sind.

Die in Form eines Ausführungsbeispiels gezeigte flächige Elektrode kann in jedem der bekannten Materialien zur Herstellung von Neutralelektroden für den einfachen oder mehrfachen Gebrauch umgesetzt werden. Insbesondere kann die leitfähige Fläche aus einer mit leitfähigem Gel oder Kleber beschichteten Metallfolie bestehen. Die linienförmige Isolierzone 8 ist z.B. 2 bis 5 mm breit und kann entweder völlig frei von leitfähigem Material, oder auch mit schwach querleitfähigem Gel oder Kleber überdeckt sein. Es können im Rahmen der Erfindung auch von diesen Angaben abweichende Bauformen als flächige Elektrode angewandt werden.

Erfindungsgemäss sind die zwei Elektrodenflächen 1, 2 durch die linienförmige Isolierzone 8 voneinander getrennt, zumindest teilweise von bandförmigen Verlängerungsabschnitten 3, 4 der jeweils anderen Elektrodenfläche umschlossen. Gegenüber den bandförmigen Verlängerungsabschnitten 3, 4, die außenliegend angeordnet sind, nehmen die zwei Elektrodenflächen 1, 2 einen zentralen inneren Bereich der erfindungsgemässen Elektrode ein.

Die bandförmigen Verlängerungsabschnitte 3, 4 sind im Vergleich zu den zentral angeordneten Elektrodenflächen 1, 2 schmal ausgebildet, d.h. ihre Länge ist deutlich grösser als ihre Breite.

Im inneren Bereich grenzen die zwei Elektrodenflächen 1, 2 an einer Seite entlang einem inneren Teilstück 9 der linienförmigen Isolierzone 8 aneinander, und die zwei Elektrodenflächen 1, 2 weisen jeweils einen bandförmigen Verlängerungsabschnitt 3, 4 auf, der sich jeweils um die andere Elektrodenfläche erstreckt.

Die bandförmigen Verlängerungsabschnitte 3, 4 der zwei Elektrodenflächen 1, 2 sind entlang der vom inneren Teilstück 9 ausgehenden äußeren Teilstücke 15, 16 der linienförmigen Isolierzone 8 um die jeweils andere Elektrodenfläche geführt.

Für weitere geometrische Überlegungen ist in Fig.1 eine Hauptachse 7 und eine Nebenachse 6 der erfindungsgemässen Elektrode eingezeichnet.

Das innere Teilstück der linienförmigen Isolierzone 8 verläuft geradlinig und erstreckt sich entlang einer Nebenachse 6 der flächigen Elektrode.

An ihrem dem inneren Teilstück 9 gegenüberliegenden Ende sind die Elektrodenflächen 1, 2 halbkreisförmig ausgebildet. Diese Enden sind von den Verlängerungsabschnitten 3, 4 durch die äußeren Teilstücke 15, 16 der linienförmigen Isolierzone 8 jeweils sichelförmig umgeben.

In dem in Fig.1 gezeigten Ausführungsbeispiel weist die erfindungsgemässe Elektrode eine ovale Form auf. Auf diese ist die Erfindung jedoch nicht beschränkt, sie kann auch in einer anderen geometrischen Form ausgeführt sein. Sie kann etwa gegenüber der in Fig.1 dargestellten Bauweise beliebig gestaucht oder gedehnt ausgeführt, also von quergeteilt über rund bis längsgeteilt aussehen. Außerdem kann der Abstand der linienförmigen Isolierzone 8 vom Rand in weiten Grenzen variiert werden.

Im Wesentlichen sind die zwei Elektrodenflächen 1, 2 um eine Achse senkrecht zur Ebene der erfindungsgemässen Elektrode, die durch den Schnittpunkt von Haupt- und Nebenachse 7, 6 verläuft zweifach-symmetrisch ausgebildet. Die Art der Symmetrie kann auch anders gestaltet sein.

Zur Abführung des Ableitstromes bzw. zur Einprägung eines Messstromes sind Anschlusslaschen 10, 11 an einem Ende eines der Verlängerungsabschnitte 3, 4 der Flächenelektroden 1, 2 und einem Übergangsbereich zwischen der anderen der Flächenelektroden 1, 2 und des anderen Verlängerungsabschnittes 3, 4 angeordnet. Die Anschlusslaschen 10, 11 erstrecken sich dabei an einer Seite der flächigen Elektrode in der gedachten Verlängerung des inneren Teilstückes 9 entlang der Nebenachse 6.

Wie weiters in Fig. 1 gezeigt erstrecken sich die Verlängerungsabschnitte 3, 4 jeweils in einem entlang der Nebenachse 6 gelegenen Eckbereich 13, 14 der Flächenelektroden 1, 2 von diesen weg, wobei die Eckbereiche 13, 14 gegenüber der Hauptachse 7 der flächigen Elektrode gleich beabstandet sind. Die bandförmigen Verlängerungsabschnitte 3, 4 verlaufen jeweils um die andere der zwei Elektrodenflächen 1, 2 bis im wesentlich in die Nähe des Eckbereiches 13, 14 dieser anderen Elektrodenfläche.

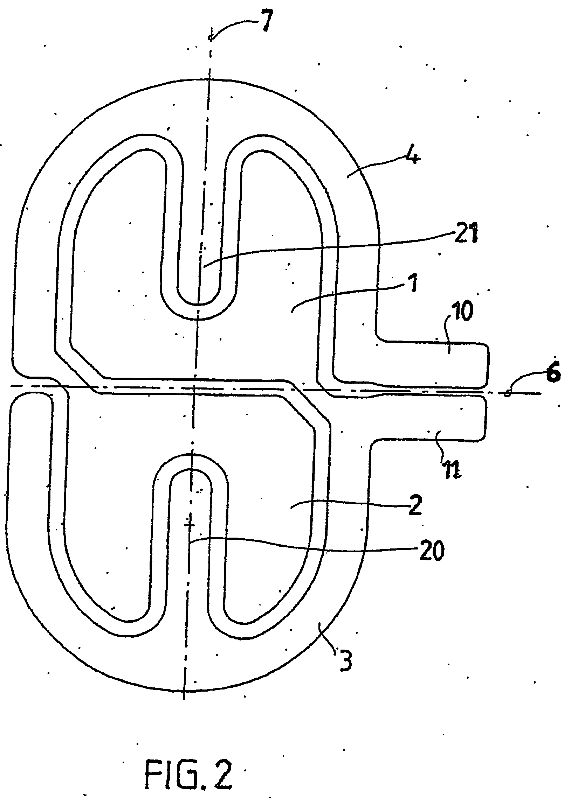

Bei der in Fig. 2 gezeigten Ausführungsform erstreckt sich jeweils entlang der Hauptachse 7 ein Vorsprung 20, 21 der bandförmigen Verlängerungsabschnitte 3, 4 in die jeweils andere Elektrodenfläche 1, 2 und verlängert dadurch die linienförmige Isolierzone 8.

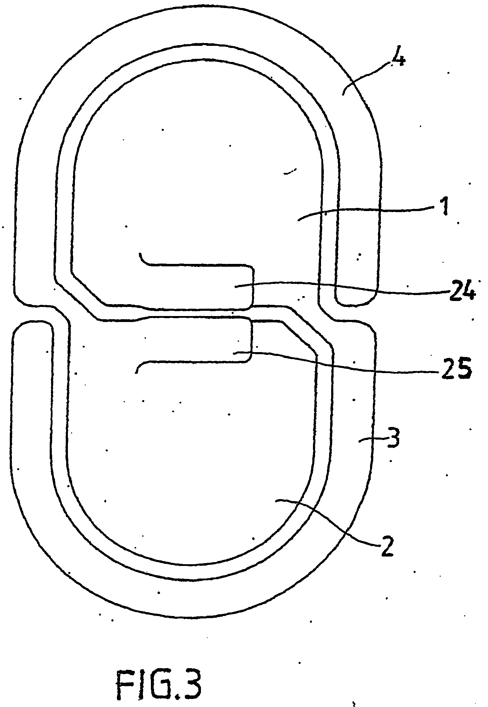

Der mittlere Bereich der erfindungsgemässen Elektrode nimmt aufgrund des Randeffektes einen vergleichsweise geringen Stromanteil auf und weist daher einen geringeren Wirkungsgrad auf als die Randbereiche. In der in Fig. 3 gezeigten Ausführungsform sind daher platz- und materialsparend Anschlusslaschen 24, 25 in diesem mittleren Bereich vorgesehen, welche mit den Elektrodenflächen 1, 2 verbunden sind.

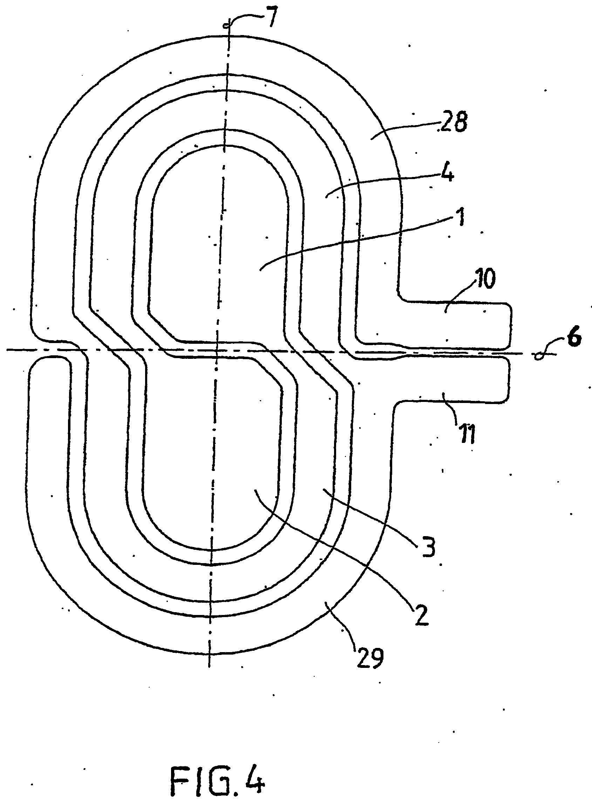

Schließlich sind in der in Fig. 4 angegebenen Ausführungsform der erfindungsgemässen Elektrode die um die jeweils andere Elektrodenfläche 1, 2 geführten Verlängerungsabschnitte 3, 4 anschließend in den Bereich der zugehörigen Elektrodenfläche zurückgeführt und umgeben die jeweils anderen Verlängerungsabschnitte 3, 4 in Form von weiter außen liegender Abschnitte 28, 29.

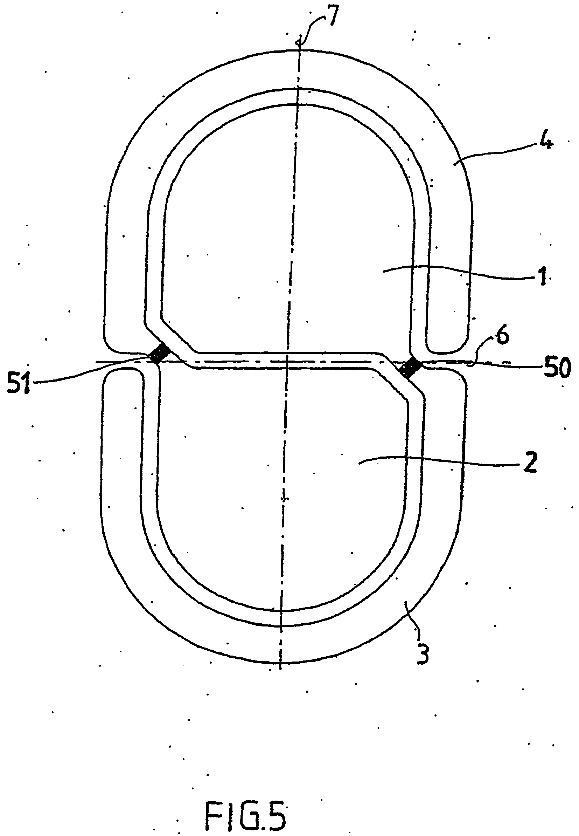

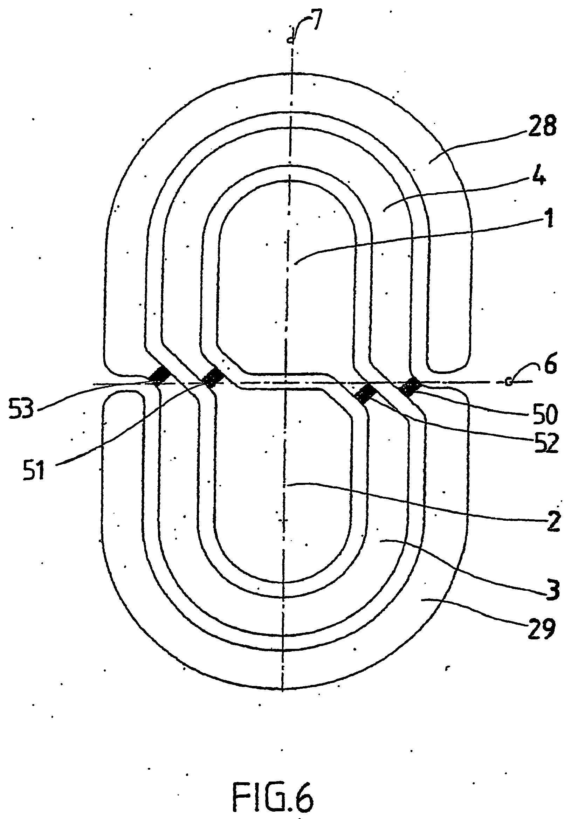

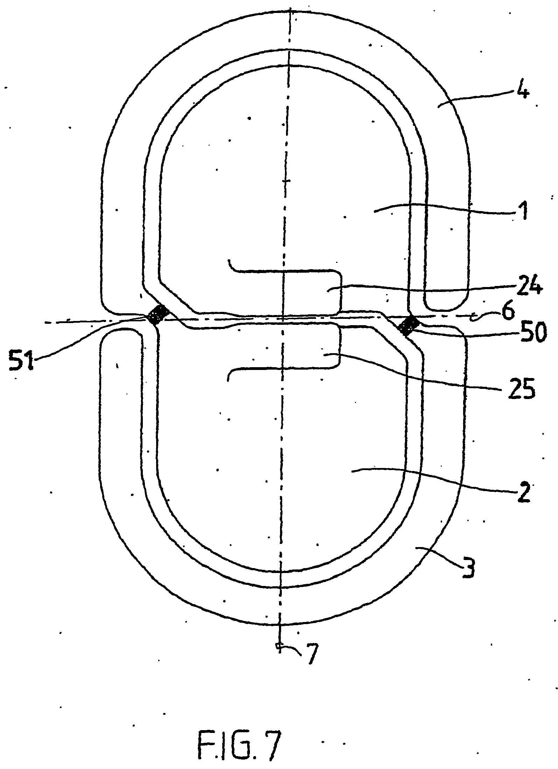

Fig.5, 6 und 7 zeigen weitere Ausführungsbeispiele der Erfindung, bei denen die Anschlusslaschen 24, 25 (Fig.7) mittig angeordnet sind. In den Fig. 5 und 6 ist die Anbringung der Anschlusslaschen nicht gezeigt, weil sie auch auf eine andere Art als der in Fig. 7 gezeigten geschehen kann, dies aber nichts zur Erfindung beiträgt.

In den Elektroden gemäß Fig. 5, 6 und 7 sind entlang des Verlaufes der bandförmigen Verlängerungsabschnitte 3, 4 diese an einer oder mehreren Stellen unterbrochen und Impedanzen 50, 51, 52, 53 zur Ausbildung eines Impedanzgradienten zwischengeschaltet. Die Anordnung der Impedanzen kann z.B. so geschehen, dass die Nebenachse 6 durch die Anbringungsstellen der Impedanzen 50, 51, 52, 52 verläuft, wodurch eine schrittweise Erhöhung der Impedanz nach außen hin verwirklicht werden kann. Dadurch lässt sich die Stromverteilung innerhalb der erfindungsgemässen Elektrode beeinflussen. Die Art, die Dimensionierung und die Anbringungsstellen der Impedanzen 50, 51, 52, 53 kann je nach Bedarf variiert werden.

Die Impedanzen 50, 51, 52, 53 selbst können sowie die Elektrodenflächen 1, 2 und die Verlängerungsabschnitte 3, 4 bzw. die außenliegenden Verlängerungsabschnitte 28, 29 durch Aufdrucken von mehr oder weniger leitfähigen Lacken aufgedruckt werden.

## Patentansprüche

1. Flächige Elektrode, insbesondere Neutralelektrode, für medizinische Zwecke mit zwei gleich großen, entlang einer linienförmigen Isolierzone (8) elektrisch voneinander getrennten Elektrodenflächen (1, 2), die entlang einem inneren Randteilbereich unmittelbar aneinandergrenzen,
- wobei die zwei gleich großen Elektrodenflächen (1 ,2) jeweils einen bandförmigen Verlängerungsabschnitt (3, 4) aufweisen, welcher die jeweils andere Elektrodenfläche (1, 2) zumindest entlang einem äußeren Randteilbereich (15, 16) umgibt und dabei durch die verlängerte Isolierzone (8) von der jeweils umgebenen Elektrodenfläche (1, 2) getrennt ist,
- wobei die bandförmigen Verlängerungsabschnitte (3, 4) außenliegend angeordnet sind und die zwei gleich großen Elektrodenflächen (1, 2) gegenüber den bandförmigen Verlängerungsabschnitten (3, 4) einen zentralen inneren Bereich der flächigen Elektrode einnehmen,
- wobei die beiden äußeren Randteilbereiche (15, 16) sich im Wesentlichen jeweils ausgehend vom einen Ende des inneren Randteilbereiches (9) um die jeweils andere Elektrodenfläche (1, 2) herum zumindest zum gegenüberliegenden anderen Ende des inneren Randteilbereiches (9) erstrecken,
- und wobei sich jeweils entlang einer Hauptachse (7) ein Vorsprung (20, 21) der bandförmigen Verlängerungsabschnitte (3, 4) in die jeweils andere Elektrodenfläche (1, 2) erstreckt.

2. Flächige Elektrode nach Anspruch 1, **dadurch gekennzeichnet, dass** ein inneres Teilstück (9) der linienförmigen Isolierzone (8) und die aneinandergrenzenden inneren Randteilbereiche der zwei gleich großen Elektrodenflächen (1, 2) geradlinig verlaufen und sich entlang einer Nebenachse (6) der flächigen Elektrode erstrecken.

3. Flächige Elektrode nach Anspruch 1 oder 2, **dadurch gekennzeichnet, dass** die zwei gleich großen Elektrodenflächen (1, 2) an ihrem dem inneren Teilstück (9) der linienförmigen Isolierzone (8) gegenüberliegenden Ende halbkreisförmig ausgebildet sind, welches Ende von den bandförmigen Verlängerungsabschnitten (3, 4) durch die äußeren Teilstücke (15, 16) der linienförmigen Isolierzone (8) jeweils sichelförmig umgeben ist.

4. Flächige Elektrode nach Anspruch 1, 2 oder 3, **dadurch gekennzeichnet, dass** die zwei Elektrodenflächen (1, 2) um eine Achse senkrecht zur Ebene der flächigen Elektrode zweifach-symmetrisch ausgebildet sind.

5. Flächige Elektrode nach einem der vorhergehenden Ansprüche 1 bis 4, **dadurch gekennzeichnet, dass** Anschlusslaschen (10, 11) an einem Ende eines der Verlängerungsabschnitte (3, 4) der Flächenelektroden (1, 2) und einem Übergangsbereich zwischen der anderen der Flächenelektroden (1, 2) und des anderen Verlängerungsabschnittes (3, 4) angeordnet sind.

6. Flächige Elektrode nach Anspruch 5, **dadurch gekennzeichnet, dass** die Anschlusslaschen (10, 11) sich an einer Seite der flächigen Elektrode in der gedachten Verlängerung des inneren Teilstückes (9) entlang der Nebenachse (6) erstrecken.

7. Flächige Elektrode nach einem der vorhergehenden Ansprüche, **dadurch gekennzeichnet, dass** die Verlängerungsabschnitte (3, 4) sich jeweils in einem entlang der Nebenachse (6) gelegenen Eckbereich (13, 14) der Flächenelektroden (1, 2) von diesen wegerstrecken, wobei die Eckbereiche (13, 14) gegenüber der Hauptachse (7) der flächigen Elektrode gleich beabstandet sind, und dass die Verlängerungsabschnitte (3, 4) sich jeweils um die andere der zwei Elektrodenflächen (1, 2) bis im Wesentlichen in die Nähe des Eckbereiches (13, 14) der anderen Elektrodenfläche erstrecken.

8. Flächige Elektrode, insbesondere nach einem der Ansprüche 1 bis 4, **dadurch gekennzeichnet, dass** Anschlusslaschen (24, 25) im mittleren Bereich vorgesehen sind, die mit den Elektrodenflächen (1, 2) verbunden sind.

9. Flächige Elektrode nach Anspruch 1, **dadurch gekennzeichnet, dass** die um die jeweils andere Elektrodenfläche (1,2) geführten Verlängerungsabschnitte (3, 4) anschließend in den Bereich der zugehörigen Elektrodenfläche (1, 2) zurückgeführt sind und dort, getrennt durch die linienförmige Isolierzone (8), die jeweils anderen Verlängerungsabschnitte (3, 4) umgeben.

10. Flächige Elektrode nach Anspruch 1, **dadurch gekennzeichnet, dass** die bandförmigen Verlängerungsabschnitte (3, 4) um die beiden Elektrodenflächen (1, 2) spiralförmig ineinanderverlaufend zwei oder mehrfach herumgeführt sind.

11. Flächige Elektrode nach einem der vorhergehenden Ansprüche, **dadurch gekennzeichnet, dass** entlang des Verlaufes der bandförmigen Verlängerungsabschnitte (3, 4) diese an einer oder mehreren Stellen unterbrochen und Impedanzen (50, 51, 52, 53) zur Ausbildung eines Impedanzgradienten zwischengeschaltet sind.

12. Flächige Elektrode nach Anspruch 11, **dadurch gekennzeichnet, dass** die Nebenachse (6) durch die Anbringungsstellen der Impedanzen (50, 51 , 52, 53) verläuft.

13. Flächige Elektrode nach Anspruch 1, **dadurch gekennzeichnet, dass** sich der Vorsprung (20, 21) der bandförmigen Verlängerungsabschnitte (3, 4) jeweils so in die jeweils andere Elektrodenfläche (1, 2) erstreckt, dass eine erhöhte Länge der Isolierzone vorliegt.

## Claims

1. Planar electrode, in particular a neutral electrode, for medical purposes with two equally sized electrode areas (1, 2) which are electrically separated from each other along a linear insulation zone (8), which electrode areas adjoin directly along an inner edge portion,
wherein each of the two equally sized electrode areas (1, 2) comprises a band-shaped extension section (3, 4) which encloses the respective other electrode area (1, 2) at least along an outer edge portion (15, 16) and is thereby separated by the extended insulation zone (8) from the respective enclosed electrode area (1, 2),
wherein the band-shaped extension sections (3, 4) are arranged outside and the two equally sized electrode areas (1, 2) take, opposed to the band-shaped extension sections (3, 4), a central inner region of the planar electrode,
wherein each of the outer edge portions (15, 16) substantially extends from one end of the inner edge portion (9) around the respective other electrode area (1, 2) at least to the opposed other end of the inner edge portion (9),
and wherein a protrusion (20, 21) of the band-shaped extension sections (3, 4) extends along a main axis (7) into the respective other electrode area (1, 2).

2. Planar electrode according to claim 1, **characterized in that** an inner part (9) of the linear insulation zone (8) and the adjoining inner edge portions of the two equally sized electrode areas (1, 2) runs linearly and extends along a secondary axis (6) of the planar electrode.

3. Planar electrode according to claim 1 or 2, **characterized in that** the two equally sized electrode areas (1, 2) are formed semi-circular at their end, which end is opposed to the inner portion (9) of the linear insulation zone (8), which end is enclosed by the band-shaped extension sections (3, 4) through the outer portions (15, 16) of the linear insulation zone (8), respectively in a crescent-shaped manner.

4. Planar electrode according to claim 1, 2 or 3, **characterized in that** the two electrode areas (1, 2) are formed double-symmetrically around an axis which is perpendicular to the plane of the planar electrode.

5. Planar electrode according to one of the preceding claims 1 to 4, **characterized in that** terminal lugs (10, 11) are arranged at an end of one of the extension sections (3, 4) of the planar electrode (1, 2) and at a transition region between the other one of the planar electrodes (1, 2) and the other extension section (3, 4).

6. Planar electrode according to claim 5, **characterized in that** the terminal lugs (10, 11) extend at a side of the planar electrode in the imaginary extension of the inner portion (9) along a secondary axis (6).

7. Planar electrode according to one of the preceding claims, **characterized in that**
the extension sections (3, 4) respectively extend in a corner region (13, 14) of the planar electrodes (1, 2) which corner region (13, 14) is located along the secondary axis (6) away from these planar electrodes (1, 2),
wherein the corner regions (13, 14) are equally spaced opposed to the main axis (7) of the planar electrode, and
the extension sections (3, 4) respectively extend around the other one of the two electrode areas (1, 2) substantially into the proximity of the corner region (13, 14) of the other electrode area.

8. Planar electrode, in particular according to one of the claims 1 to 4, **characterized in that** terminal lugs (24, 25), which are connected to the electrode areas (1, 2), are provided in the middle region.

9. Planar electrodes according to claim 1, **characterized in that**
the extension sections (3, 4), which are guided around the respective other electrode area (1, 2), are subsequently guided back to the region of the associated electrode area (1, 2) and there, separated by the linear insulation zone (8), enclose the respective other extension section (3, 4).

10. Planar electrode according to claim 1, **characterized in that** the band-shaped extension sections (3, 4) are guided around both of the electrode areas (1, 2) intertwined in a spiraliformed manner two or more times.

11. Planar electrode according to one of the preceding claims, **characterized in that**
along the course of the band-shaped extension sections (3, 4), these are interrupted at one or more positions and
impedances (50, 51, 52, 53) are interconnected for the formation of impedance gradients.

12. Planar electrodes according to claim 11, **characterized in that** the secondary axis (6) runs through the attachment position of the impedances (50, 51, 52, 53).

13. Planar electrode according to claim 1, **characterized in that** the protrusion (20, 21) of the band-shaped extension sections (3, 4) respectively extends in the respective other electrode area (1, 2) such that an increased length of the insulation zone is present.

## Revendications

1. Electrode plate, en particulier électrode neutre, à usage médical avec deux surfaces d'électrode (1, 2) de même dimension séparées électriquement l'une de l'autre le long d'une zone d'isolation (8) de forme linéaire, qui sont directement adjacentes le long d'une portion de bordure interne,
- **caractérisée en ce que** les deux surfaces d'électrode (1, 2) de même dimension présentent respectivement un segment de prolongement (3, 4) en forme de bande qui entoure l'autre surface d'électrode (1, 2) respective au moins le long d'une portion de bordure externe (15, 16) et, ce faisant, est séparé de la surface d'électrode (1, 2) respectivement entourée par la zone d'isolation (8) prolongée,
- **caractérisée en ce que** les segments de prolongement (3, 4) en forme de bande sont agencés à l'extérieur et les deux surfaces d'électrode (1, 2) de même dimension en face des segments de prolongement (3, 4) en forme de bande occupent une zone interne centrale de l'électrode plate,
- **caractérisée en ce que** les deux portions de bordure externes (15, 16) s'étendent essentiellement respectivement en partant d'une extrémité de la portion de bordure interne (9) autour de l'autre surface d'électrode (1, 2) respective au moins jusqu'à l'autre extrémité opposée de la portion de bordure interne (9),
- et **caractérisée en ce qu'**une saillie (20, 21) des segments de prolongement (3, 4) en forme de bande s'étend respectivement dans l'autre surface d'électrode (1, 2) respective le long d'un axe principal (7).

2. Electrode plate selon la revendication 1, **caractérisée en ce qu'**une section interne (9) de la zone d'isolation (8) de forme linéaire et les portions de bordure internes adjacentes des deux surfaces d'électrode (1, 2) de même dimension font une ligne droite et s'étendent le long d'un axe secondaire (6) de l'électrode plate.

3. Electrode plate selon la revendication 1 ou 2, **caractérisée en ce que** les deux surfaces d'électrode (1, 2) de même dimension sont formées semi-circulaires au niveau de leur extrémité opposée à la section interne (9) de la zone d'isolation (8) de forme linéaire, laquelle extrémité est entourée respectivement sous la forme d'une faucille par les segments de prolongement (3, 4) en forme de bande par l'intermédiaire des sections externes (15, 16) de la zone d'isolation (8) de forme linéaire.

4. Electrode plate selon la revendication 1, 2 ou 3, **caractérisée en ce que** les deux surfaces d'électrode (1, 2) sont formées de façon doublement symétrique autour d'un axe perpendiculairement au plan de l'électrode plate.

5. Electrode plate selon l'une des revendications précédentes 1 à 4, **caractérisée en ce que** des pattes de raccordement (10, 11) sont agencées au niveau d'une extrémité de l'un des segments de prolongement (3, 4) des électrodes de surface (1, 2) et au niveau d'une zone de transition entre l'autre des électrodes de surface (1, 2) et l'autre segment de prolongement (3, 4).

6. Electrode plate selon la revendication 5, **caractérisée en ce que** les pattes de raccordement (10, 11) s'étendent au niveau d'un côté de l'électrode plate dans le prolongement imaginaire de la section interne (9) le long de l'axe secondaire (6).

7. Electrode plate selon l'une des revendications précédentes, **caractérisée en ce que** les segments de prolongement (3, 4), dans une zone d'angle (13, 14) des électrodes plates (1, 2) située le long de l'axe secondaire (6), s'éloignent respectivement de celles-ci, **caractérisée en ce que** les zones d'angle (13, 14) sont à équidistance par rapport à l'axe principal (7) de l'électrode plate, et **en ce que** les segments de prolongement (3, 4) s'étendent respectivement autour de l'autre des deux surfaces d'électrode (1, 2) sensiblement jusqu'au voisinage de la zone d'angle (13, 14) de l'autre surface d'électrode.

8. Electrode plate, en particulier selon l'une des revendications 1 à 4, **caractérisée en ce que** des pattes de raccordement (24, 25) sont prévues dans la zone médiane, qui sont reliées aux surfaces d'électrode (1, 2).

9. Electrode plate selon la revendication 1, **caractérisée en ce que** les segments de prolongement (3, 4) amenés autour de l'autre surface d'électrode (1, 2) respective sont ensuite ramenés dans la zone de la surface d'électrode (1, 2) correspondante, et là, séparés par la zone d'isolation (8) de forme linéaire, entourent les autres segments de prolongement (3, 4) respectifs.

10. Electrode plate selon la revendication 1, **caractérisée en ce que** les segments de prolongement (3, 4) en forme de bande sont amenés autour des deux surfaces d'électrode (1, 2) en s'entrelaçant en spirale deux fois ou plus.

11. Electrode plate selon l'une des revendications précédentes, **caractérisée en ce que** le long du parcours des segments de prolongement (3, 4) en forme de bande, ceux-ci sont interrompus en un ou plusieurs endroits et des impédances (50, 51, 52, 53) sont intercalées pour former un gradient d'impédance.

12. Electrode plate selon la revendication 11, **caractérisée en ce que** l'axe secondaire (6) passe à travers les emplacements des impédances (50, 51, 52, 53).

13. Electrode plate selon la revendication 1, **caractérisée en ce que** la saillie (20, 21) des segments de prolongement (3, 4) en forme de bande s'étend respectivement dans l'autre surface d'électrode (1, 2) respective de telle sorte que la zone d'isolation présente une longueur plus importante.
